# EUROPEAN PATENT APPLICATION

(11) **EP 1 306 664 A1**
(43) Date of publication of application: **02.05.2003**
(21) Application number: 01917540.5
(22) Date of filing: 28.03.2001
(51) Int. Cl.: G01N 21/49, D21F 7/00, G01B 11/26, G01N 33/34

(54) **METHOD FOR MEASURING ORIENTATION OF PAPER FIBERS AND APPARATUS FOR MEASURING ORIENTATION OF PAPER FIBERS**

(30) Priority: 30.03.2000 JP 2000094457
(71) Applicant: NIPPON PAPER INDUSTRIES CO., LTD., Kita-ku, Tokyo 114-0002 (JP)
(72) Inventor: FUKUOKA, Kazuhiko, c/o NIPPON PAPER IND CO LTD, Iwakuni-shi, Yamaguchi 740-0003 (JP); TODOROKI, Hidenobu, c/o NIPPON PAPER IND CO LTD, Kita-ku, Tokyo 114-0002 (JP); NURUKI, Yutaka, c/o NIPPON PAPER INDUSTRIES CO LTD, Kita-ku, Tokyo 114-0002 (JP); ABE, Yuji, c/o NIPPON PAPER IND CO LTD, Kita-ku, Tokyo 114-0002 (JP)
(74) Representative: Zipse + Habersack
(86) International application number: JP0102562
(87) International publication number: WO01075423

(57) **Abstract**

The present invention aims to provide method and apparatus to measure paper fiber orientation adapted to measure fiber orientation in this paper from an image to be measured formed by light beam transmitted through paper on the paper side opposite to the incident side thereof improved so that the fiber orientation in this paper being running through a paper making machine can be reliably on-line measured.

Paper web (W) is irradiated practically at a right angle thereto with light beam emitted from a pulse laser source (1) and unpolarized through a condenser lens (2) and an image to be measured formed on a surface of the paper web (W) opposite to its incident surface is picked up by a CCD camera (3). An image pick-up optical system (3b) of the CCD camera (3) uses an extra depth-of-field lens. The image picked up in this manner is then image processed for elliptic approximation. Then a fiber orientation angle relative to a major axis of the obtained ellipse and a magnitude of fiber orientation based on a difference or a ratio between the major and minor axes of this ellipse are calculated. Pick-up by the CCD camera (3) may be carried out at predetermined periods and with an appropriate pulse width to pick up a target image as if the paper web (W) is in stationary state.

## Description

### TECHNICAL FIELD OF THE INVENTION

The present invention relates to method and apparatus for measurement of fiber orientation and particularly to such method and apparatus suitable for measurement of fiber orientation in paper during a process of paper making.

### DESCRIPTION OF THE RELATED ART

To make paper of high quality, it is essential to determine paper quality as well as fiber orientation of the paper obtained as finished product. Various apparatuses for this purpose have already been proposed. German Patent Application Disclosure Gazette No. 34134558A1 discloses a fiber orientation measuring apparatus of this type adapted to detect a differential magnitude of two extreme values of fiber orientation, using a diffusion effect (i.e., light guide effect). This fiber orientation measuring apparatus of well known art non-contact detects any extreme fiber orientation appearing in roll paper, particularly running roll paper and/or detects a differential magnitude of two such extreme fiber orientations. In this case, the paper web is irradiated with electromagnetic radiation having a sharp boundary over at least 180° in its cross-section, particularly the laser beam of visible spectrum (visible light). Then, in the vicinity of incidence spot on the incidence side of the paper web or on the side opposed thereto, the beam transmitted through the paper web is divided into a plurality of predetermined sectors, measured at the position at a predetermined distance from the boundary between the incidence spot and non-incidence region and the measured values in the form of electrical values are compared to each other. To obtain such electrical values, the light beam transmitted through the paper web is optically magnified and guided through an image pick-up lens to obtain an image which is then subjected to photoelectric conversion.

The well known method and apparatus to measure the paper fiber orientation as has been described above is based on a principle such that a detector is rotated around the optical axis of the incident laser beam to divide this into a plurality of predetermined shapes over the given angle and thereby to obtain a difference or a ratio between the maximum value and the minimum value. However, such method and apparatus have been accompanied with various problems as follow. First, it may be impossible to detect the maximum value and the minimum value at one and same position on the paper web being made since the web is running during rotation of the detector. In order that such apparatus of well known art can achieve the desired measurement, it is essential that the paper fiber orientation to be determined should be constant at any position on the paper web. Regrettably, the fiber orientation more or less depend on the particular position on the paper web being made and therefore it may be impossible for this apparatus of well known art to detect the fiber orientation being variable as the paper web runs. Thus, it may be thus substantially impossible to on-line determine the fiber orientation of the web running through the paper making machine. In other words, it may be impossible to reflect the result of measurement instantaneously in paper being made and thereby to obtain paper having desired fiber orientation.

Additionally, the paper web may be affected by variable running velocity as well as variable diameter of wind-up roll and may shake and/or vibrate in a direction substantially perpendicular to its surface. Consequently, it is difficult to maintain the distance between the paper web and the image pick-up lens constant and thereby to maintain the image in well focused condition. Distinctness as well as shape of the image in the imaging plane may vary due to shaking and/or vibration of the web. Such variation may not be related to an actual variation in the fiber orientation and the intensity of the light beam determined by the detector at a predetermined position for measurement may vary independently of the actual fiber orientation. There is an anxiety that the fiber orientation in the paper web being made could not be adjusted to a desired value due to apprehension that accurate fiber orientation could not be detected and the result of measurement might be instable.

In view of the problems as have been described above, it is a principal object of the present invention to provide method and apparatus to measure paper fiber orientation improved so that the fiber orientation in the paper web being running can be reliably on-line measured and thereby the result of measurement can be instantaneously reflected on paper being made in order to obtain paper of high quality.

### DISCLOSURE OF THE INVENTION

The object set forth above is achieved, according to one aspect of this invention, a method adapted to measure fiber orientation in paper by irradiating said paper with focused light beam and picking up the light transmitted through said paper, said method comprising steps of: unpolarizing said focused light beam substantially to present a approximately true circle cross-section and irradiating said paper practically at a right angle thereto with such focused light beam, picking up a range to be measured by image pick-up means, said range including an image to be measured formed by the light beam transmitted through said paper on a surface of said paper opposite to its incident surface, conditioning the image picked up by said image pick-up means to a predetermined image reflecting fiber orientation in said paper by image processor means, and calculating said image reflecting fiber orientation to obtain fiber orientation of said paper.

Upon irradiation of paper with the light beam emitted from said projector means, an image to be measured reflecting particular fiber orientation in said paper form by the light beam transmitted through paper appears on a paper surface opposite to its incident surface. Specifically, the incident light beam entering paper fiber is guided in the fiber under a light guide effect, then diffused in the direction of this fiber and the diffused light beam appears on the paper surface opposite to its incident surface. For example, when non-oriented paper is irradiated with the focused light beam having true circle-shaped cross-section, the image to be measured also presents a true circle-shape. In the case of paper more or less oriented, the image to be measured presenting a shape reflecting such fiber orientation appears and this image to be measured presents a flattened circle-shape. This image to be measured reflects the fiber orientation in the entire layer of paper since this image has been transmitted through this paper.
Upon irradiation of paper with the focused light beam, an image to be measured reflecting particular fiber orientation in said paper form by the light beam transmitted through paper appears on a paper surface opposite to its incident surface. Specifically, the incident light beam entering paper fiber is guided in the fiber, then diffused in the direction of this fiber and the diffused light beam appears on the paper surface opposite to its incident surface. For example, when non-oriented paper is irradiated with the focused light beam having true circle-shaped cross-section, the image to be measured also presents a true circle-shape. In the case of paper more or less oriented, the image to be measured presenting a shape reflecting such fiber orientation appears and this image to be measured presents a flattened circle-shape. This image to be measured reflects the fiber orientation in the entire layer of paper since this image has been transmitted through this paper.

This image to be measured is picked up by said image pick-up means. A range to be picked up should be dimensioned to be sufficiently large to cover this image to be measured and the image to be measure should have a distinct outer peripheral edge. The image to be measured thus picked up is binarized by said image processor means and therefore the flattened circle is approximated to an ellipse. A direction of a major axis of this ellipse approximated from the image reflecting the particular fiber orientation is measured with respect to the machine direction of the paper making machine. Lengths of the major and minor axes are measured and a magnitude of fiber orientation is obtained from difference or ratio of said lengths of the major and minor axes of the ellipse.

The image to be measured picked up by the image pick-up means is the image formed by the light beam transmitted through paper, so said image to be measured can be instantaneously caught and the fiber orientation can be obtained on the basis of the image data. In other words, the fiber orientation in paper being made can be on-line obtained. Therefore, it is possible to reflect the fiber orientation obtained in this manner on paper being made without a delay and thereby to make paper having a desired fiber orientation.

The method to measure fiber orientation in paper according to Claim 2 is characterized by that said focused light beam is stroboscopic light beam having light emission period shorter than a period given in second depending on a running speed of said paper and said image pick-up means picks up said range to be measured in synchronization with the light emission period of this stroboscopic light beam and the method to measure fiber orientation in paper according to Claim 3 is characterized by that said focused light beam is continuous light beam and said image pick-up means picks up said range to be measured within time duration shorter than a time duration giver in second shorter than time duration depending on the running speed of said paper at predetermined periods.

Thus, said image to be measured is picked up at regular periods and the fiber orientation is measured at regular time intervals. The light emission period or the time duration of each image pick-up may be set to be relatively short to pick up the image to be measured practically in stationary state even when the measuring pick-up is carried out on paper running through the paper making machine.

The apparatus to measure fiber orientation in paper according to Claim 4 irradiates said paper with focused light beam and picks up the light transmitted through said paper, wherein said apparatus comprising projector means adapted to unpolarize said focused light beam substantially to present a approximately true circle cross-section and then to irradiate said paper practically at a right angle thereto with such focused light beam, image pick-up means adapted to pick up a range to be measured by image pick-up means, said range including an image to be measured formed by the light beam transmitted through said paper on a surface of said paper opposite to its incident surface, image processor means adapted to condition the image picked up by said image pick-up means to a predetermined image reflecting fiber orientation in said paper and calculating means adapted to calculate fiber orientation in said paper from said image reflecting the fiber orientation in said paper obtained by said image processor means.

Upon irradiation of paper with the light beam emitted from said projector means, an image to be measured reflecting particular fiber orientation in said paper form by the light beam transmitted through paper appears on a paper surface opposite to its incident surface. Specifically, the incident light beam entering paper fiber is guided in the fiber, then diffused in the direction of this fiber and the diffused light beam appears on the paper surface opposite to its incident surface. For example, when non-oriented paper is irradiated with the focused light beam having true circle-shaped cross-section, the image to be measured also presents a true circle-shape. In the case of paper more or less oriented, the image to be measured presenting a shape reflecting such fiber orientation appears and this image to be measured presents a flattened circle-shape. This image to be measured reflects the fiber orientation in the entire layer of paper since this image has been transmitted through this paper.

The image to be measured picked up by the image pick-up means is binarized by said image processor means and thereby the flattened circle is approximated to an ellipse. In said calculating means, the fiber orientation angle is determined on the basis of a direction of the major axis in this approximated ellipse reflecting the fiber orientation and the magnitude of the fiber orientation is determined from difference or ratio between lengths of the major and minor axes of the ellipse.

The image to be measured picked up by the image pick-up means is the image formed by the light beam transmitted through paper, so said image to be measured can be instantaneously caught and the fiber orientation can be obtained on the basis of the image data. In other words, the fiber orientation in paper being made can be on-line obtained. Therefore, it is possible to reflect the fiber orientation obtained in this manner on paper being made without a delay and thereby to make paper having a desired fiber orientation.

The apparatus to measure fiber orientation in paper according to Claim 5 is characterized by that said projector means comprises a stroboscopic source adapted to emit stroboscopic light beam for light emission period shorter than that giver in second depending on the running speed of said paper; and said image pick-up means is adapted to pick up said range to be measured in synchronization with the light emission period of said stroboscopic source and the apparatus to measure fiber orientation in paper according to Claim 6 is characterized by that said projector means is adapted to emit continuous light beam so as to irradiate said paper in continuous fashion; and said image pick-up means is adapted to pick up said range to be measured within time duration given in second shorter than time duration depending on the running speed of said paper at predetermined periods.

Thus, said image to be measured is picked up at regular periods and the fiber orientation is measured at regular time intervals. The light emission period or the time duration of each image pick-up may be set to be relatively short to pick up the image to be measured practically in stationary state even when the measuring pick-up is carried out on paper running through the paper making machine.

The apparatus to measure fiber orientation in paper according to Claim 7 is characterized by that a CCD camera is used as said image pick-up means.

Use of the CCD camera enables this fiber orientation measuring apparatus to be miniaturized.

The apparatus to measure fiber orientation in paper according to Claim 8 is characterized by that said image pick-up means includes an image pick-up optical system having an optical magnification of 0.2 ∼ 2.0 and being acceptably focusable even a distance from an object to be picked up to an objective lens varies by 30 %.

A distance from the paper web to the image pick-up means varies as the paper web running through the paper making machine shakes and/or vibrates. As a result, the image to be measured may be out of focus. To overcome this problem, the image pick-up optical system may be incorporated with an extra depth-of-field lens to ensure a relatively large object distance over which the image can be well focused. For example, the depth of field of image pick-up optical system is set to 2 mm in back and forth direction so that a variation in the distance between the paper web and the image pick-up means may be reliably followed. Such image pick-up optical system ensures that the image pick-up means can maintain its focusing state and the fiber orientation can be reliably measured even if the paper web running through the paper making machine shakes and/or vibrates.

Use of the condenser lens having a long focal distance also enables a variation in a distance between the paper web and the projector means to be reliably followed. This is for the reason that a long focal distance alleviates a variation in the outer diameter of the focused light beam in the direction of optical axis. Specifically, even if the paper web shakes and/or vibrates and moves from the focused position, the outer diameter of the focused light beam entering said paper web is substantially free from variation and therefore the shape of the incident light beam can be maintained practically constant.

The apparatus to measure fiber orientation in paper according to Claim 9 is characterized by that said projector means uses a condenser lens having a relatively long focal distance adapted to alleviate a variation of the focused light beam's outer diameter in the direction of optical axis possibly occurring on the focal spot.

For example, the condenser lens is preferably used, which can maintain the outer diameter of the focused light beam substantially in a constant dimension so far as shaking and/or vibration of the paper web is limited within a range of 2 mm in back and forth direction.

The apparatus to measure fiber orientation in paper according to Claim 10 is characterized by that optical fiber is used in said projector means so as to guide the light beam emitted from the source approximately at a right angle to said paper and thereby to irradiate said paper with said light beam.

If the semiconductor laser source is used, its emitter will take a rectangular form having a relatively large aspect ratio. The laser beam emitted such source may be guided through the optical fiber to obtain a circular spot with an appropriately small diameter for irradiation of paper. It should be understood that the exit beam from the optical fiber is preferably focused by a lens system before entering the paper.

The apparatus to measure fiber orientation in paper according to Claim 11 is characterized by that a product (d × NA) of a diameter d (µm) and a numerical aperture NA is less than 300 µm.

If a diameter (d) of exit beam from the optical fiber is relatively large, a lens system having a short focal distance is required to obtain a desired fine spot. However, if focusing is too sharp, there is an anxiety that the paper web might shift out of the spot of a desired diameter even when the paper web shakes and/or vibrates and slightly moves in the direction of optical axis. Also when the optical fiber having a relatively high diffusivity, i.e., a large numerical aperture (NA) is used, there is the same anxiety that the paper web might shift out of the spot of a desired diameter even when the paper web shakes and/or vibrates and slightly moves in the direction of optical axis. To overcome this problem, it is desirable to limit a product (d × NA) of these two factors to a predetermined value or lower and thereby to reconcile the fine focal spot and the versatile focusing.

### BRIEF DESCRIPTION OF THE DRAWINGS

Fig. 1 is a block diagram schematically illustrating the paper fiber orientation measuring apparatus according to a first embodiment of the invention.
Fig. 2 is a block diagram illustrating the picked up image processing system in the paper fiber orientation measuring apparatus according to the first embodiment of the invention.
Fig. 3 is a graphic diagram comparatively plotting the fiber orientation ratios obtained by the fiber orientation measuring apparatus according to the first embodiment of the invention and by the conventional molecular orientation meter.
Fig. 4 is a graphic diagram comparatively plotting the fiber orientation angles obtained by the fiber orientation measuring apparatus according to the first embodiment of the invention and by the conventional molecular orientation meter.
Fig. 5 is a block diagram schematically illustrating the paper fiber orientation measuring apparatus according to a second embodiment of the invention.
Fig. 6 is a diagram schematically illustrating an experimental apparatus used to conduct the measurement with the fiber orientation measuring apparatus according to the invention on the assumption that the paper web shakes during the measurement.
Fig. 7 is a graphic diagram plotting the fiber orientation ratios (major axis length/minor axis length) obtained using an ordinary lens as the image pick-up lens for the image pick-up means of the paper fiber orientation measuring apparatus according to the invention on the assumption that the paper web shakes during the measurement.
Fig. 8 is a graphic diagram plotting the fiber orientation ratios (major axis length/minor axis length) obtained using a sufficiently high depth-of-field lens as the image pick-up lens for the image pick-up means of the paper fiber orientation measuring apparatus according to the invention on the assumption that the paper web shakes during the measurement.
Fig. 9 is a block diagram schematically illustrating the paper fiber orientation measuring apparatus according to a third embodiment of the invention.
Fig. 10 is a graphic diagram plotting the fiber orientation angles obtained using the paper fiber orientation measuring apparatus according to the third embodiment of the invention on the paper web which is running.
Fig. 11 is a graphic diagram plotting the fiber orientation ratios obtained using the paper fiber orientation measuring apparatus according to the third embodiment of the invention on the paper web which is running.
Fig. 12 is a graphic diagram comparatively plotting the fiber orientation angles obtained using the paper fiber orientation measuring apparatus according to the third , embodiment of the invention on the paper web running at a speed of 200 m/min, on one hand, and the fiber orientation angles obtained by off-line measurement using the molecular orientation meter, on the other hand.
Fig. 13 is a graphic diagram comparatively plotting the fiber orientation angles obtained using the paper fiber orientation measuring apparatus according to the third embodiment of the invention on the paper web running at a speed of 1000 m/min, on one hand, and the fiber orientation angles obtained by off-line measurement using the molecular orientation meter, on the other hand.
Fig. 14 is a graphic diagram comparatively plotting the fiber orientation ratios obtained using the paper fiber orientation measuring apparatus according to the third embodiment of the invention on the paper web running at a speed of 200 m/min, on one hand, and the fiber orientation ratios obtained by off-line measurement using the molecular orientation meter, on the other hand.
Fig. 15 is a graphic diagram comparatively plotting the fiber orientation ratios obtained using the paper fiber orientation measuring apparatus according to the third embodiment of the invention on the paper web running at a speed of 1000 m/min, on one hand, and the fiber orientation ratios obtained by off-line measurement using the molecular orientation meter, on the other hand.

### PREFERRED EMBODIMENTS OF THE INVENTION

The method and the apparatus for measurement of paper fiber orientation according to the present invention will be more fully understood from the description of the preferred embodiments given hereunder in reference with the accompanying drawings.

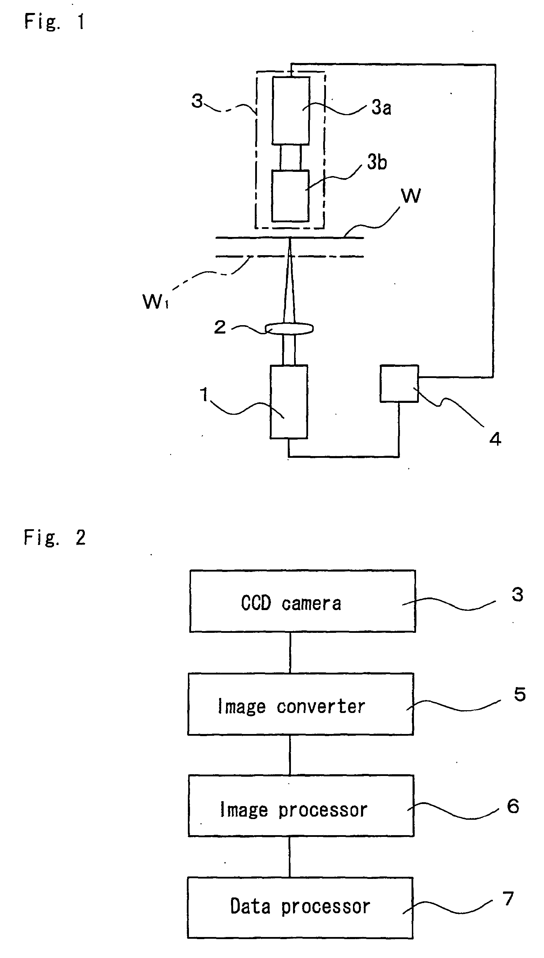

Fig. 1 is a block diagram schematically illustrating the paper fiber orientation measuring apparatus according to the invention. A pulse laser source 1 serving as projector means is provided to face one surface of paper web W of which the fiber orientation are to be determined so that the paper web W may be irradiated with a laser beam emitted from this pulse laser source 1. The laser beam emitted from this pulse laser source 1 is substantially circular unpolarized laser beam. In front of the pulse laser source 1, a condenser lens 2 is located in front of the pulse laser source 1 to condense the laser beam emitted from this source 1 and thereby to adjust a diameter of the laser beam appropriately to enter the paper web W.

On the side of the paper web W opposed to the laser beam incident side thereof, a CCD camera 3 is provided as image pick-up means. This CCD camera 3 includes an image pick-up device 3a located on the side of the paper web W opposed to the laser beam incident side thereof to pick up a target image formed by the incident laser beam having been transmitted through the paper web W at the position opposite to the incident point. It should be understood that this CCD camera 3 is appropriately adjusted to have a range of field for image pick-up inclusive of the target image. This range of field for image pick-up corresponds to a range of fiber orientation measurement. An image pick-up optical system of this CCD camera 3 adopts an image pick-up lens comprising an extra depth-of-field lens having a depth of field of 2 mm or more.

Said pulse laser source 1 is connected to a pulse width modulator 4 to which said CCD camera 3 is connected so that an internal synchronizing signal provided from said CCD camera 3 is input to the pulse width modulator 4 and said pulse laser source 1 emits the laser beam in accordance with said synchronizing signal.

Image data relating to the target image which has been picked up, as will be apparent in Fig. 2, by said CCD camera is transmitted to an image modulator 5 in which said image data is subjected to various processing such as A/D conversion and then output from said image modulator 5 to an image processor 6. In this image processor 6, the laser beam is ellipse-approximated on the basis of the image data relating to the target image. The laser beam emitted from the pulse laser source 1 is intrinsically unpolarized beam substantially of truly circular cross-section and, also after transmitted through the paper web, the laser beam has such cross-section so far as any significant orientation angle is not present. However, if any significant fiber orientation angle is present, the circular cross-section of the laser beam becomes more or less flattened. This flattened circular cross-section is ellipse-approximated by said image processor 6 and converted to the image reflecting the fiber orientation. In this way, the orientation can be steadily detected.

Data relating to such fiber orientation reflecting image ellipse-approximated by said image processor 6 is then applied to a data processor 7 in which a predetermined processing including a series of calculation is carried out. Specifically, an angle of the major axis of the image reflecting the fiber orientation with respect to the direction in which the paper web runs is calculated as the fiber orientation angle and a differential length or a ratio of the major axis and the minor axis is calculated as the fiber orientation magnitude. Furthermore, a group of the fiber orientation angles and a group of the fiber orientation magnitudes obtained within a predetermined time are averaged, respectively. The data calculated in this manner are displayed on a display device such as CRT display in the form of graphs or numeric data. If it is desired, the data may be written into an external memory or output from a printer.

The paper fiber orientation measuring apparatus according to the above-mentioned embodiment of the present invention operates in a manner as will be described.
The paper web W is irradiated with the unpolarized and truly circular cross-sectioned laser beam emitted from said pulse laser source 1 at a predetermined period under control of an output signal from said pulse width modulator 4. After condensed by said condenser lens 2 to have an appropriate diameter, the laser beam enter the paper web W. This laser beam is partially reflected on the incidence surface of the paper web W and partially transmitted through the paper web W. The laser beam penetrates into the fiber as the laser beam is transmitted through the paper web W. Thereupon, the fiber functions like optical fiber and the laser beam is guided and propagated under a light guide effect of the fiber along a direction in which the fiber extends. The laser beam transmitted through the paper web W to side opposite to the incidence side forms an image having a shape which reflects particular fiber orientation. This image formed by the transmitted laser beam is the image to be determined and picked up by the CCD camera 3. The CCD camera 3 has a range of field sufficient to cover said image to be determined and an outer peripheral edge of this image to be determined can be distinctly detected.

The image pick-up optical system 3b of the CCD camera 3 uses an image pick-up lens having a relatively high depth-of-field. It is thereby ensured that, even if a distance between the plane of the paper web W and the image pick-up plane of the CCD camera 3 is not constant, the image to be determined can be sharply picked up without any focal shift in said image pick-up plane. The image to be determined is carried out in non-interlacing mode by this CCD camera 3 and the internal synchronizing signal output from said CCD camera 3 is input to said pulse width modulator. After appropriately pulse width modulated in said pulse width modulator, said internal synchronizing signal is input to the pulse laser source 1. Consequently, the pulse laser source 1 emits the laser beam in synchronization with the timing of image acquisition by the CCD camera 3. In other words, the CCD camera 3 picks up the image to be determined at the emission timing of the laser beam, independently of a shutter speed. Thus the image to be determined which has been formed by the transmitted laser beam can be reliably picked up the CCD camera 3 without being affected by the ambient light or the like.
Data of the image to be determined which has been picked up by the CCD camera 3 are transmitted to said image converter 5 in which the data are subjected to predetermined processing such as A/D conversion and then output to said image processor 6. The image processor 6 convert to the image reflecting the fiber orientation, for example, by ellipse-approximating the image to be determined which has been picked up using elliptic function and the data of the image obtained in this manner is transmitted to said data processor 7.

Said data processor 7 calculates a fiber orientation angle by measuring an angle included between a major axis direction of the ellipse reflecting said fiber orientation and a given direction, for example, the paper making machine direction, and this fiber orientation angle is stored in an internal memory or the like. The data processor 7 operates also to determine a fiber orientation magnitude by calculating a difference or a ratio between a major axis length and a minor axis length of the ellipse and this fiber orientation magnitude is also stored in the internal memory or the like. Said CCD camera 3 picks up the images to be determined at predetermined periods, so that the fiber orientation angle and magnitude are calculated at said predetermined periods and successively stored in the internal memory or the like. The data relating to these fiber orientation angles and magnitudes stored during a given time are displayed on the appropriate display device such as CRT display in the form of numeric data or graph and, if necessary, written into an external memory and/or printed by a printer.

The invention will be understood more in details from comparison of the measurement data obtained using the paper fiber orientation measuring apparatus according to the present embodiment to the corresponding measurement data using the paper fiber orientation measuring apparatus of well known art. The result of this comparison will be described hereunder.

Four sheets of paper made by an orienting sheet machine and having a basis weight of 100g/m² were used as samples for measurement of the fiber orientation. Each of these four samples was dimensioned to be of 100 mm × 100 mm. Laser beam emitted from a He-Ne laser source 1 was transmitted through a beam attenuator filter and attenuated to 103µW. A molecular orientation meter MOA-2001A manufactured by Kanzaki Paper Co., Ltd. (present Oji Paper Co., Ltd.) was used as the fiber orientation determining apparatus of well known art. This molecular orientation meter is adapted to determine the fiber orientation of paper's entire layer using absorption of microwave and commercially available. Concerning the range of measurement, the fiber orientation measuring apparatus according to the present embodiment uses a condenser lens 2 to form an image of substantially ϕ1 mm to be determined. On the other hand, irradiation beam used by said molecular orientation meter has a diameter larger than the diameter of the image to be determined by the fiber orientation measuring apparatus according to the present embodiment. In order to ensure accuracy of the measured value to be compared, the fiber orientation measuring apparatus according to the present embodiment irradiated 24 regions per sample sheet, each region dimensioned to be approximately 25 mm × 15 mm, and acquired the measured value for each of the samples by averaging measured values in 25 regions.

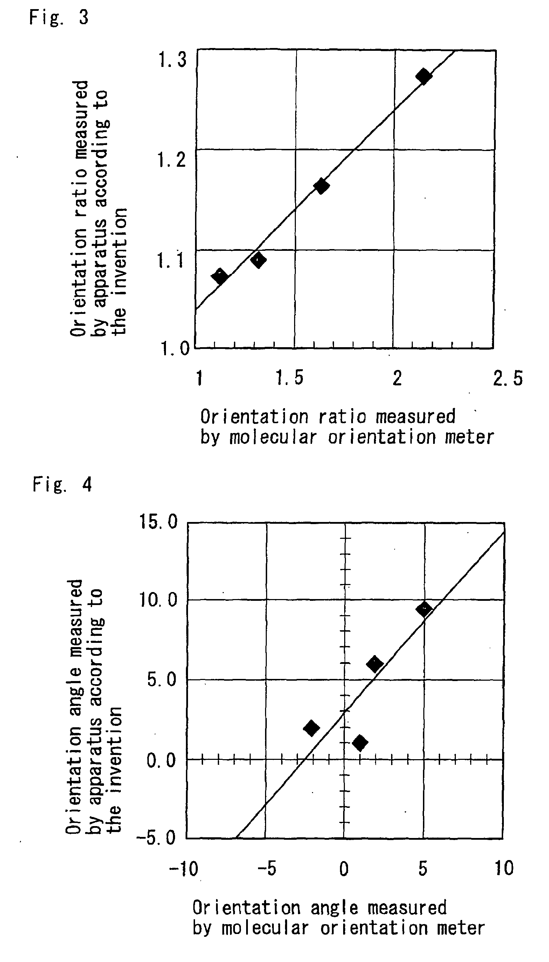

The result of the comparative measurement is shown in Figs. 3 and 4. Fig. 3 relates to the fiber orientation magnitude ratio (major axis length/minor axis length) and Fig. 4 relates to the fiber orientation angle, wherein the measured value obtained by the molecular orientation meter is indicated on the axis of abscissa and the measured value obtained by the fiber orientation measuring apparatus is indicated on the axis of ordinates. As will be apparent from Figs. 3 and 4, the measured value obtained by the fiber orientation measuring apparatus according to the present embodiment is correlated with the measured value obtained by the molecular orientation meter.

Now a manner in which the fiber orientation is measured as the paper web W runs on the paper making machine will be described. The CCD camera 3 picks up the image in 30Hz non-interlace mode and the internal synchronizing signal of 30Hz output from the CCD camera 3 is input to the pulse laser source 1 after appropriately pulse width modulated by the pulse width modulator 4 whereupon said pulse laser source 1 irradiates the paper web W with the laser beam in synchronization with the image acquisition timing of the CCD camera 3. In this manner, the CCD camera 3 picks up the image during the emission period of the laser beam.
Assumed that the emission period is 10µec and the paper web W runs at a paper making speed, for example, of 1000 m/min, the paper web W will move by 0.17 mm while the image to be measured is being picked up under emission of the laser beam and a dimension of the image picked up in this manner will be correspondingly reduced with respect to said size of the image to be measured (ϕ1 mm). Consequently, the measurement can be carried out as if the paper web W is substantially in stationary state even when the paper web W is running at a relatively high speed. Measurement conducted on paper web made by the oriented sheet machine and having a basis weight of 100g/m² indicated that the output of the pulse laser source 1 may be adjusted in a range of 1.5µJ ∼ 12.4µJ to conduct the measurement. Specification of the apparatus used for this measurement is as follows:
CCD camera: XC-55 manufactured by SONY, which operates in progressive scan mode.
YAG laser: Beam diameter of 2.5 mm, stroboscopic light emission period (pulse width) of 7 nm, laser emission period of 30 Hz.
Focal distance of the condenser lens: 180 mm.

Measurement conducted on paper having a basis weight of several ten g/m² ∼ one hundred several ten g/m² such as paper used for newspaper or postcard requires the source 1 to have an output of several µJ ∼ several ten µJ.
While the case in which the paper web is made at the speed of 1000 mm/min and the laser emission period of 10 µsec has been described above, it is also possible at the paper making speed of 2000 mm/min to pick up the image as if it is a stationary image. This is for the reason that the paper web moves by 0.34 mm during the laser emission period and a size of the picked up image is sufficiently reduced with respect to said size of the image to be measured (ϕ1 mm). Assumed that the laser emission period is set to 5 µsec, the paper web will move by 0.17 mm during this period and the measurement will be carried out in the same manner as in the case of the paper making speed set to 1000 mm/min. Namely, the emission period of laser beam may be set to a period in seconds inversely proportional to the running speed of the paper web, or a shorter period.

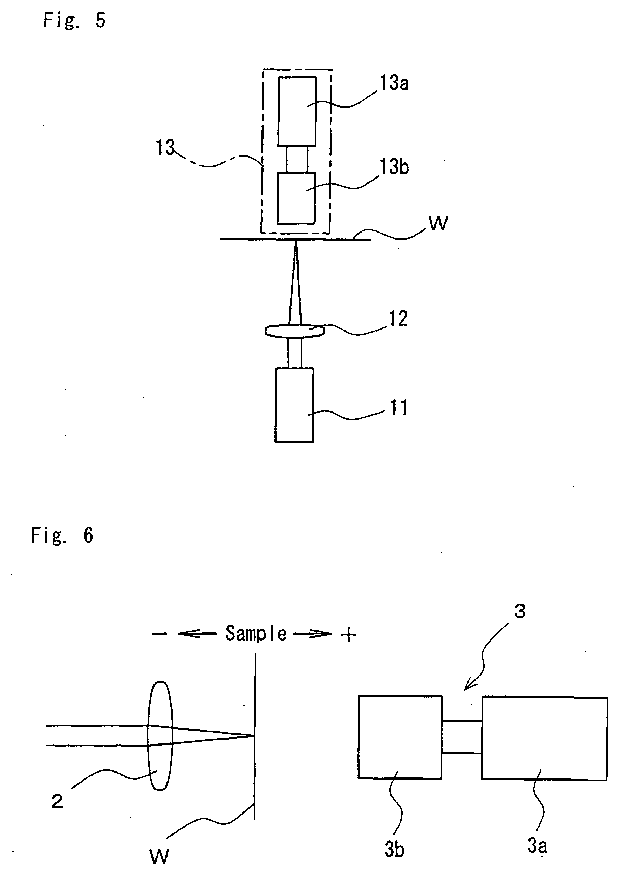

Fig. 5 is a block diagram schematically illustrating the paper fiber orientation measuring apparatus according to a second embodiment of the invention. In opposition to one surface of the paper web W of which the fiber orientation is to be measured, there is provided a continuous laser source 11 serving as projector means adapted to emit laser beam in continuous mode and, in front of the continuous laser source 11, there is provided a condenser lens 12 adapted to condense the laser beam emitted from the continuous laser source 11 and then to irradiate the paper web W with this laser beam along a direction substantially orthogonal to the paper web W. Opposed to the incidence side of the laser beam with the paper web W therebetween, there is provided a CCD camera 13 serving as image pick-up means comprising an image pick-up device 13a and an image pick-up optical system 13 which includes, in turn, an image pick-up lens having a relatively large depth-of-field. The image pick-up optical system 13b further includes a shutter device adapted to be opened and closed at predetermined periods.

In the case of this fiber orientation measuring apparatus according to the second embodiment, the paper web W is irradiated with the continuous laser beam emitted from the laser source 11 and an image to be measured is picked up by the CCD camera 13 at predetermined periods. Said pulse width modulator 4 used in the first embodiment is not required in this second embodiment since the image to be measured is picked up by the CCD camera 13 at a desired shutter speed to which said shutter device may be set. The shutter speed may be set to 10µsec or less to ensure that the image can be picked up as if the paper web is in stationary state as has been described with respect to the first embodiment. In the case of this second embodiment also, the shutter speed may be set to a speed in seconds depending on the running speed of the paper web, or a lower speed.

On the assumption that the paper web W is shaking and/or vibrating in the course of measurement, focusing accuracy in the CCD camera 3 was comparatively determined and the result of this determination will be described hereunder. Fig. 6 is a diagram schematically illustrating an experimental apparatus used to conduct this determination. As illustrated, the paper web W is irradiated with the laser beam condensed by the condenser lens 2 and, in opposition to the incidence side of the laser beam, there is provided the CCD camera 3 comprising the image pick-up optical system 3b and the image pick-up device 3a. The fiber orientation was measured with the paper web W being moved in the direction substantially orthogonal to the laser beam. For this measurement, a He-Ne laser was used as the continuous laser source 11 and the paper web W was irradiated with the laser beam transmitted through the condenser lens 2 having a focal distance of 180 mm. As samples for the paper web W, remarkably oriented paper sheets made by the oriented sheet machine and having a basis weight of 72 g/m² were used.
For comparison, a commonly used lens and a high depth-of-field lens were used as the image pick-up lenses in the respective CCD camera 3. As the high depth-of-field lens, extra depth-of-field lens VDF-EDF manufactured by RAM OPTICAL INSTRUMENTATION INC. was used. He-Ne laser of ϕ3 mm was emitted from the continuous laser source 11 and the paper web W was irradiated with the laser beam which had been transmitted through the condenser lens 2.

The CCD camera 3 was placed at distance of approximately 35 mm from the object to be image picked up and the position of said paper web W at which an image to be measured is focused by said CCD camera 3 was set as a reference position. The paper web W as moved back and forth with respect to this reference position by 0.5 mm, respectively, and the fiber orientation was measured at these positions, respectively. The position at which the paper web W is irradiated with the laser beam was maintained constant.

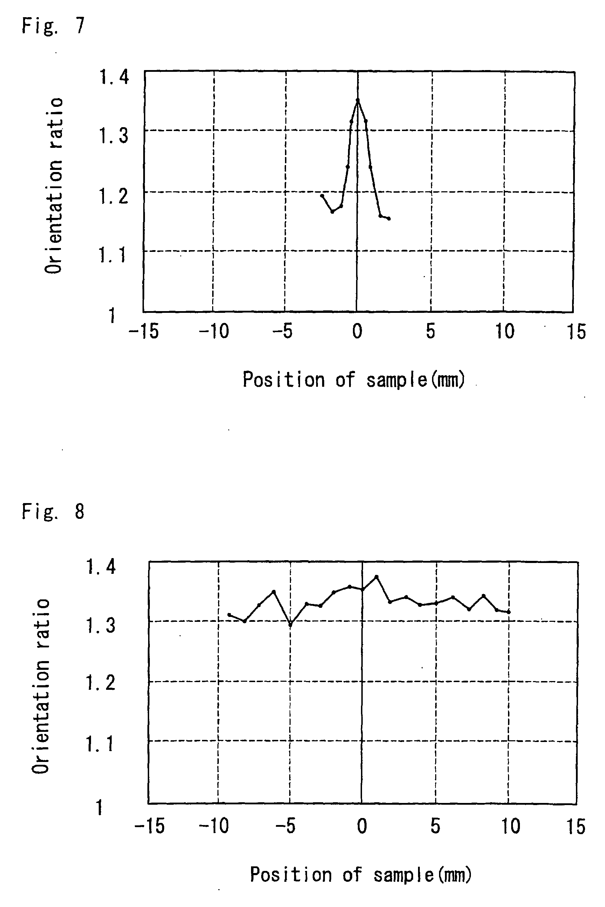

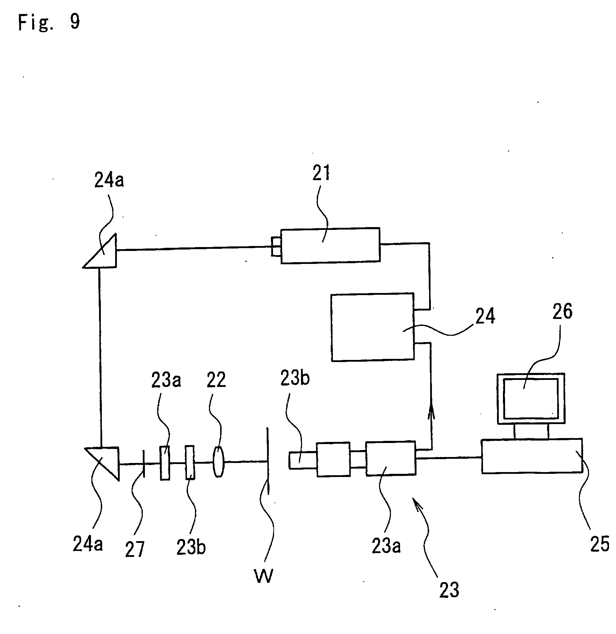

The result of this measurement is shown by Figs. 7 and 8, wherein Fig. 7 shows the result obtained with use of the commonly used image pick-up lens and Fig. 8 shows the result obtained with use of the extra depth-of-field lens. As will be apparent from Fig. 7, the maximum fiber orientation ratio of approximately 1.35 was obtained at the reference position (0 mm) and this value decreased as the position of the paper web W moves back and forth from said reference position. This was due to a phenomenon that the picked up image was out of focus and was deformed to the true circle. The image to be measured was unacceptably out of focus as the position of the paper web W moved back and forth from the reference position by 2.5 mm or more and the effective measurement was no more possible.

With use of the extra depth-of-field lens, as will be apparent from Fig. 8, a constant fiber orientation ratio of approximately 1.35 was obtained in a range of 5.0 mm back and forth from the reference position and, based on this result, it is believed that no focal shift occurs even when the position of the paper web W moves.
As will be apparent from such result of comparative measurement, use of the extra depth-of-field as the image pick-up lens in the CCD camera 3 enables the image to be measured to be reliably picked up even if the paper web W running on the paper making machine is shaking and/or vibrating in the course of the measurement and thereby enables a variation possibly occurring due to such shaking and/or vibration to be avoided.

Preferably, the paper web W is irradiated with the laser beam of a diameter as small as possible. However, even if the laser beam is condensed prior to irradiation, shaking and/or vibration of the paper web W may result in movement of its position along the optical path of the laser beam to a position on said optical path represented by W₁ as indicated by an imaginary line in Fig. 1. If the laser beam has not sufficiently condensed at the position of the paper web W₁, the laser beam of a diameter too large to form a desired image will enter the paper web W₁ and make a steady measurement impossible. To overcome this problem, the laser beam is preferably guided through not only said condenser lens 2, 12 but also an appropriate adjusting optical system to obtain parallel beam of desired small diameter which enters the paper web W. More specifically, the diameter of the parallel beam being incident on the paper web W is invariable even if the paper web W shakes and/or vibrates and moves along the optical path of said parallel beam. In this way, it is possible to irradiate the paper web W with the laser beam of an invariable diameter and thereby to obtain the image to be measured which can be used for the steady measurement.

Using a test coater, the fiber orientation of the paper web W running therethrough was measured. Fig. 9 is a block diagram schematically illustrating the paper fiber orientation measuring apparatus according to a third embodiment of the invention which was used for this measurement. A condenser lens 22 is placed so as to face one surface of sample paper web W and a CCD camera 23 is placed so as to face the opposite surface of the sample paper web W. The laser beam successively passes through a pinhole of a diaphragm 27, a beam attenuating filter 28a and 1/4 wavelength constant 28b and then enters the condenser lens 22. The beam attenuating filter 28a is adjusted to a beam intensity depending on a basis weight of the sample paper web W and the 1/4 wavelength constant 28b is adapted to convert polarized light of the laser beam from linearly polarized light to circularly polarized light (unpolarized light). In the case of this third embodiment, the laser beam is emitted from a pulse laser source 21 and guided by appropriate optical systems 24a, 24b to said diaphragm 27. Said CCD camera 23 also comprises the image pick-up device 23a and the image pick-up optical system 23b. This image pick-up optical system 23b includes an extra depth-of field lens adapted to maintain a desired focused condition even if the paper web W is shaking and/or vibrating. Said pulse laser source 21 is applied with a light emission control signal output from a pulse width modulator 24, with which the light emission is controlled. The pulse width modulator 24 is applied, in turn, with an internal synchronizing signal output from said CCD camera23, with which the light emission control signal is transmitted to the pulse laser source 21. The image picked up by said CCD camera 23 is input to an image processor 25 basically comprising a computer in which the image is appropriately processed, and the result of processing is displayed on a display device 26 such as CRT display or printed by a printer (not shown). If it is desired, the data may be written into an external memory or output from a printer. Similarly to the previous embodiments, the image picked up is averaged, binarized and thereby elliptic approximation. An angle included between a major axis of the ellipse obtained by said approximation and the machine direction is determined as the fiber orientation angle and a ratio between the major and minor axes of the ellipse is determined as the fiber orientation ratio.

Specification of the devices illustrated by Fig. 9 in the block diagram is as follows:
Pulse laser source 21: Mine Lase II Nd: YAG Laser Systems manufactured by NEW WAVE RESEARCH Corp. Pulse frequency of 30Hz, Pulse width of 7 nsec.
Condenser lens 22: Focal distance of 380 mm
   Light attenuating filter 28a: ND50 % (Basis weight less than 45 g/m²)
CCD camera 23: XC-55 manufactured by SONY
Extra depth-of-field lens (23b): VDF-EDF manufactured by RAM OPTICAL
INSTRUMENTATION, INC. Magnification: approximately 1.1
   Image processor 25: Cobra/C6 manufactured by CORECO Corp. Object-to-lens distance was set to 10 ∼ 12 mm with focusable range of 3 ∼ 4 mm.

Measurement was conducted on various samples of paper as follows:

| | |
|---|---|
| (1) Base paper for postcard | 165 g/m² mixed with waste paper |
| (2) Base paper for ink jet | 85g/m² fine quality |
| (3) Base paper to be coated | 44.5g/m² medium quality |
| (4) Base paper to be coated | 48.9g/m² medium quality |
| (5) Base paper to be coated | 57.6g/m₂ fine quality |
| (6) Base paper to be coated | 117.0g/m² fine quality |
| (7) PPC paper | 80g/m² fine quality |
| (8) Newspaper(super light) | 43g/m² mixed with waste paper |
| (9) Newspaper(supersuper light) | 40g/m² mixed with waste paper |

The other conditions for measurement were as follows:
(1) Machine through which the paper web runs: Test coater
(2) Running speed: 200, 1000 m/min
(3) Position of measuring: Web center
(4) Measuring period: 1 min

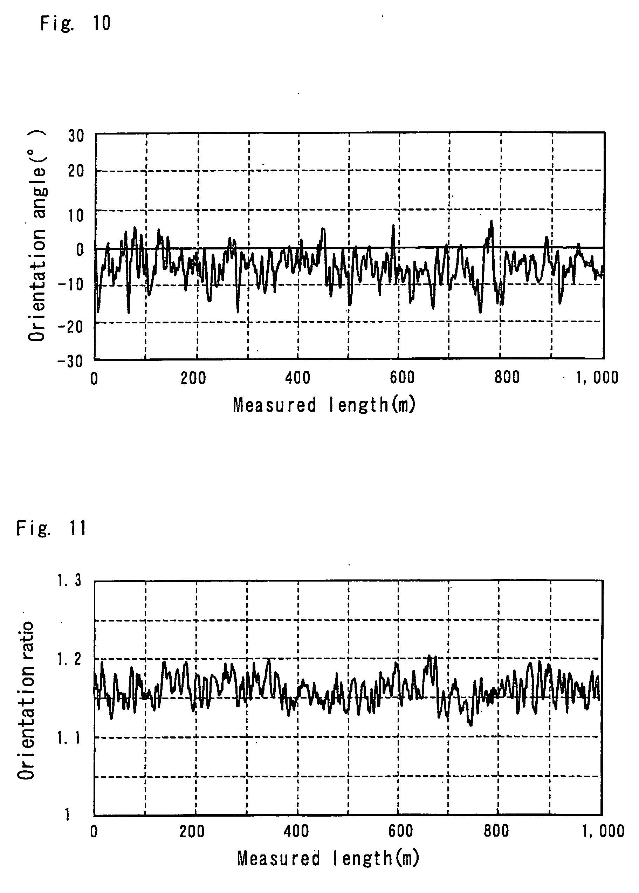

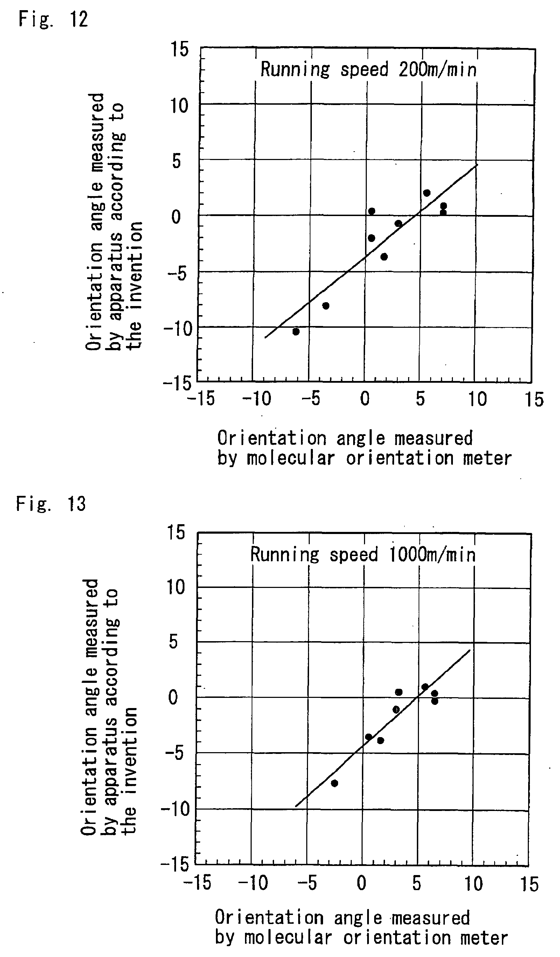

Under the condition as set forth above, the laser beam having a pulse width of 7 nsec was emitted from the pulse laser source 21 at 30Hz in synchronization with the image pick-up by the CCD camera 23 occurring for 1/30 sec to obtain the image. The laser beam was attenuated by the light attenuating filter 28a, then converted by the 1/4 wavelength constant 28b to the unpolarized light and condensed by the condenser lens 22 so that the paper web W may be irradiated with the laser beam having a spot diameter less than 0.5 mm and a light intensity of 5 µJ ∼ 10µJ. A depth of field of said extra depth-of-field lens was set to ± 2 mm and the image formed by the beam transmitted through the sample paper web W was picked up by the CCD camera 23. The measurement was conducted for 60 sec and 1000 or more data were obtained. These data for each sample paper web were averaged to obtain the orientation value of this particular sample paper web W. The result of measurement conducted on said PPC paper having the basis weight of 80 g/m² as indicated as the sample (7) is shown in Figs. 10 and 11. This measurement was carried out by irradiating the paper web W running at the speed of 1000 m/min on wire with the laser beam. Fig. 10 shows the fiber orientation angle and Fig. 11 shows the fiber orientation ratio.

Fig. 12 is a graphic diagram comparatively plotting the fiber orientation angles obtained on the paper web running at a speed of 200 m/min on the axis of ordinate and the fiber orientation angles obtained by off-line measurement using the molecular orientation meter on the axis of abscissa. Fig. 12 suggests that the values obtained by these two different apparatuses are well correlated with each other.

Fig. 13 is a graphic diagram comparatively plotting the fiber orientation angles obtained on the paper web running at a speed of 1000 m/min on the axis of ordinate and the fiber orientation angles obtained by off-line measurement using the molecular orientation meter on the axis of abscissa. Fig. 13 also suggests that the values obtained by these two different apparatuses are well correlated with each other.

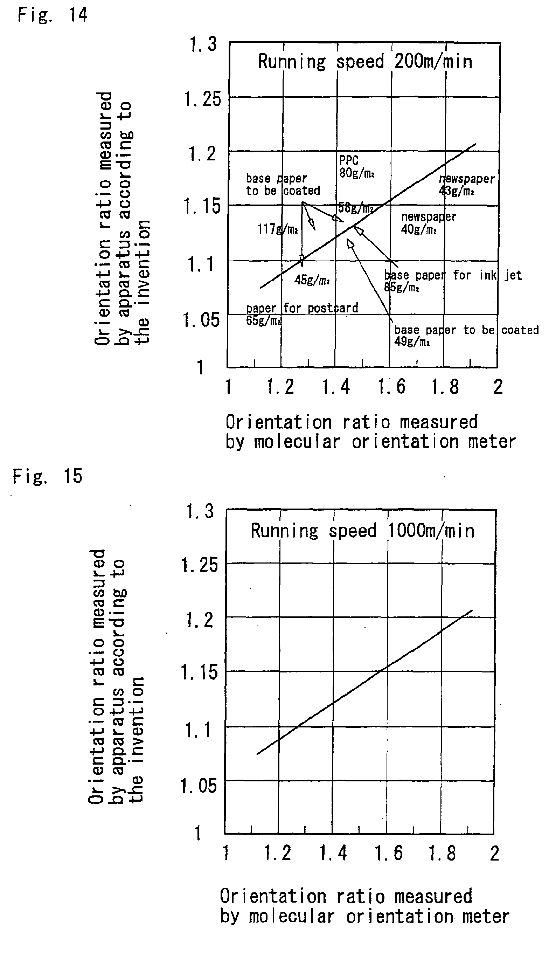

Fig. 14 is a graphic diagram comparatively plotting the fiber orientation ratios obtained on the paper web running at a speed of 200 m/min on the axis of ordinate and the fiber orientation ratios obtained by off-line measurement using the molecular orientation meter on the axis of abscissa. Fig. 14 also suggests that the values obtained by these two different apparatuses are well correlated with each other.

Fig. 15 is a graphic diagram comparatively plotting the fiber orientation ratios obtained on the paper web running at a speed of 1000 m/min on the axis of ordinate and the fiber orientation ratios obtained by off-line measurement using the molecular orientation meter on the axis of abscissa. Fig. 15 also suggests that the values obtained by these two different apparatuses are well correlated with each other.

While the present invention has been described with respect to the embodiments in which the He-Ne laser source or the YAG laser source is used as the laser source, it is possible without departing from the scope of the invention to use, for example, a semiconductor laser source. Use of the semiconductor laser is preferable to miniaturize the fiber orientation measuring apparatus. While the CCD camera set to 1/30 sec in the previously mentioned embodiments, the CCD camera of a higher speed may be used to improve accuracy of the fiber orientation measurement.

In the embodiments as have been described above, the optical systems 24a, 24b are used to form the desired optical path along which the laser beam enters the paper web and thereby to minimize the apparatus. The optical systems 24a, 24b may be replaced by optical fiber to improve a degree of freedom for selection of the optical path and to further improve miniaturization of the apparatus. Additionally, use of the optical fiber makes said 1/4 wavelength constant 28b unnecessary since the polarized light becomes random. Particularly when the semiconductor laser is used as the laser source, use of the optical fiber is desired to adjust a spot shape formed by the laser beam. Furthermore, a product of a diameter (d) of the optical fiber and a numerical aperture (NA) is preferably less than 300 µm in order to ensure that the paper web does not shift from the spot diameter even if the paper web shakes and/or vibrates.

### INDUSTRICAL APPLICABILITY

As will be apparent from the foregoing description, the method or the apparatus for measurement of the fiber orientation is adapted to irradiate the paper web of which the fiber orientation is to be measured with the unpolarized light having a cross-section substantially in true circle and then to pick up the image to be measured which is formed by the light transmitted through the paper web using the appropriate image pick-up means. In this way, the image to be measured can be instantaneously picked up. Based on the image data relating the measured image, the fiber orientation can be quickly acquired. Accordingly, the fiber orientation of the paper web running through the paper making machine can be obtained on-line mode. In other words, the fiber orientation data obtained can be quickly reflected on the paper web being made and thereby the paper web having the desired fiber orientation can be easily made.

The method or the apparatus for measurement of the fiber orientation in paper defined by any one of Claims 2, 3, 5 and 6 allows the image to be measured on the paper web being running to be picked up as if the paper web is in stationary state. In this way, the fiber orientation of the paper web running through the paper making machine can be reliably measured.

The apparatus for measurement of fiber orientation in paper defined by Claim 7 enables such apparatus to have its overall size as miniaturized as possible.

Finally, the apparatus for measurement of fiber orientation in paper defined by Claim 8 enables the image to be maintained in the focused state even if the paper web more or less shakes and/or vibrates as the paper web runs through the paper making machine and thereby enables the fiber orientation to be reliably determined. This is for the reason that the focusable range of the object to be picked up in the image forming plane of the image pick up device is adequately large.

The apparatus for measurement of fiber orientation defined by Claim 7 enables this apparatus to be drastically miniaturized.

The apparatus for measurement of fiber orientation defined by Claim 8 can maintain the image in well focused condition and thereby enables the fiber orientation to be reliably determined even if the paper web running through the paper making machine is shaking and/or vibrating. This is for the reason that the distance from the camera to the object at which the image is acceptably focused on the imaging plane of the image pick-up means is set to be adequately large.

## Claims

1. A method adapted to measure fiber orientation in paper by irradiating said paper with focused light beam and picking up the light transmitted through said paper, said method comprising steps of:
unpolarizing said focused light beam substantially to present a approximately true circle cross-section and irradiating said paper practically at a right angle thereto with such focused light beam;
picking up a range to be measured by image pick-up means, said range including an image to be measured formed by the light beam transmitted through said paper on a surface of said paper opposite to its incident surface;
conditioning the image picked up by said image pick-up means to a predetermined image reflecting fiber orientation in said paper by image processor means; and
calculating said image reflecting fiber orientation to obtain fiber orientation of said paper.

2. The method to measure fiber orientation in paper according to Claim 1, wherein said focused light beam is stroboscopic light beam having light emission period shorter than a period given in second depending on a running speed of said paper; and said image pick-up means picks up said range to be measured in synchronization with the light emission period of this stroboscopic light beam.

3. The method to measure fiber orientation in paper according to Claim 1, wherein said focused light beam is continuous light beam; and said image pick-up means picks up said range to be measured within time duration shorter than a time duration given in second shorter than time duration depending on the running speed of said paper at predetermined periods.

4. An apparatus to measure fiber orientation in paper by irradiating said paper with focused light beam and picking up the light transmitted through said paper, said apparatus comprising:
projector means adapted to unpolarize said focused light beam substantially to present an approximately true circle cross-section and then to irradiate said paper practically at a right angle thereto with such focused light beam;
image pick-up means adapted to pick up a range to be measured by image pick-up means, said range including an image to be measured formed by the light beam transmitted through said paper on a surface of said paper opposite to its incident surface;
image processor means adapted to condition the image picked up by said image pick-up means to a predetermined image reflecting fiber orientation in said paper; and calculating means adapted to calculate fiber orientation in said paper from said image reflecting the fiber orientation in said paper obtained by said image processor means.

5. The apparatus to measure fiber orientation in paper according to Claim 4, wherein said projector means comprises a stroboscopic source adapted to emit stroboscopic light beam for light emission period shorter than that given in second depending on the running speed of said paper; and said image pick-up means is adapted to pick up said range to be measured in synchronization with the light emission period of said stroboscopic source.

6. The apparatus to measure fiber orientation in paper according to Claim 4, wherein said projector means is adapted to emit continuous light beam so as to irradiate said paper in continuous fashion; and said image pick-up means is adapted to pick up said range to be measured within time duration given in second shorter than time duration depending on the running speed of said paper at predetermined periods.

7. The apparatus to measure fiber orientation in paper according to any one of Claims 4 through 6, wherein a CCD camera is used as said image pick-up means.

8. The apparatus to measure fiber orientation in paper according to any one of Claims 4 through 7, wherein said image pick-up means includes an image pick-up optical system having an optical magnification of 0.2 ∼ 2.0 and being acceptably focusable even a distance from an object to be picked up to an objective lens varies by 30 %.

9. The apparatus to measure fiber orientation in paper according to any one of Claims 4 through 8, wherein said projector means uses a condenser lens having a relatively long focal distance adapted to alleviate a variation of the focused light beam's outer diameter in the direction of optical axis possibly occurring on the focal spot.

10. The apparatus to measure fiber orientation in paper according to any one of Claims 4 through 9, wherein optical fiber is used in said projector means so as to guide the light beam emitted from the source approximately at a right angle to said paper and thereby to irradiate said paper with said light beam.

11. The apparatus to measure fiber orientation in paper according to Claim 10, wherein a product (d × NA) of a diameter d (µm) and a numerical aperture NA is less than 300 µm.
